(19) Europäisches Patentamt
European Patent Office
Office européen des brevets

(11) EP 0 826 668 B1

(12) FASCICULE DE BREVET EUROPEEN

(45) Date de publication et mention de la délivrance du brevet:
**30.06.1999 Bulletin 1999/26**

(51) Int Cl.6: **C07D 209/40**, A61K 7/13

(21) Numéro de dépôt: **97401911.9**

(22) Date de dépôt: **08.08.1997**

(54) **Dérivés de 2-imino-2, 3-dihydro-1H-indoles et leur utilisation dans des compositions pour la teinture des cheveux**

2-Imino-2,3-Dihydro-1H-Indolderivate und deren Verwendung in Haarfärbemittel

Derivatives of 2-imino-2,3-dihydro-1H-indole and their use in hair dyeing compositions

(84) Etats contractants désignés:
**DE ES FR GB IT**

(30) Priorité: **23.08.1996 FR 9610412**

(43) Date de publication de la demande:
**04.03.1998 Bulletin 1998/10**

(73) Titulaire: **L'OREAL**
**75008 Paris (FR)**

(72) Inventeurs:
- **Terranova, Eric**
**92600 Asnieres (FR)**
- **Fadli, Aziz**
**93150 Le Blanc-Mesnil (FR)**
- **Lagrange, Alain**
**77700 Coupvray (FR)**

(74) Mandataire: **Goulard, Sophie**
**L'OREAL-DPI**
**6 rue Bertrand Sincholle**
**92585 Clichy Cedex (FR)**

(56) Documents cités:
**EP-A- 0 428 441**
**WO-A-92/17157**
**FR-A- 2 008 797**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention est relative à de nouveaux dérivés de 2-imino-2,3-dihydro-1H-indoles, leur procédé de préparation, leur utilisation en teinture des fibres kératiniques telles que les cheveux et les procédés de teinture les mettant en oeuvre.

On a déjà proposé dans le passé de teindre les cheveux en utilisant comme coupleurs, certaines monohydroxyindoles ou monoaminoindolines, notamment dans le brevet français FR-A-2 008 797. Le brevet US-A-4 013 404 décrit l'utilisation de mono- ou diaminoindolines ou de monohydroxyindolines comme base d'oxydation ou comme coupleurs, en teinture d'oxydation des cheveux.

On connaît, par ailleurs, les colorants de la famille des indoles, en particulier le 5,6-dihydroxyindole, ainsi que leur utilisation pour la teinture des fibres kératiniques telles que les cheveux humains, notamment par les brevets français FR-A-1 133 594 et FR-A-1 166 172.

La demanderesse vient de découvrir des nouveaux dérivés de 2-imino-2,3-dihydro-1H-indoles monoalcoxylés, mono- ou dihydroxylés présentant des propriétés tinctoriales remarquables vis-à-vis des fibres kératiniques telles que les cheveux. Ces composés s'oxydent facilement en milieu alcalin et peuvent être utilisés en teinture capillaire sans éventuellement mettre en oeuvre un agent oxydant, ce qui permet d'obtenir une gamme variée de nuances plus ou moins intenses.

L'invention a pour objet de nouveaux dérivés de 2-imino-2,3-dihydro-1H-indoles ainsi que leurs sels d'addition avec un acide, de formule (I) ou (II) que l'on définira après.

Un autre objet de l'invention est constitué par l'utilisation de ces composés en teinture des fibres kératiniques.

L'invention a pour objet les compositions tinctoriales destinées à la teinture des fibres kératiniques et en particulier des cheveux humains, contenant au moins un dérivé de 2-imino-2,3-dihydro-1H-indoles de formule (I) ou (II) définies ci-après.

L'invention a également pour objet des procédés de teinture mettant en oeuvre ces composés.

D'autres objets de l'invention apparaîtront à la lumière de la description et des exemples qui suivent.

Les dérivés de 2-imino-2,3-dihydro-1H-indoles conformes à l'invention et leurs possibles formes tautomères répondent à la formule (I) ou (II) suivantes:

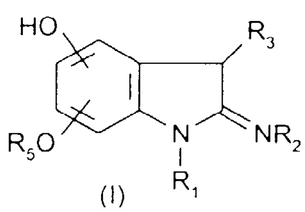
(I)
ou
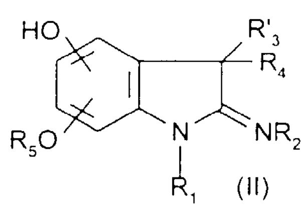
(II)

dans lesquelles:

- $R_1$, $R_2$ et $R_3$, identiques ou différents, désignent un atome d'hydrogène, un radical alkyle en $C_1$-$C_4$, carboxyle, alcoxy($C_1$-$C_4$)carbonyle, monohydroxyalkyle en $C_1$-$C_4$, polyhydroxyalkyle en $C_2$-$C_4$, alcoxy($C_1$-$C_4$)alkyle en $C_1$-$C_4$, monoalkyl($C_1$-$C_4$)aminoalkyle en $C_1$-$C_4$ ou dialkyl($C_1$-$C_4$)aminoalkyle en $C_1$-$C_4$;

- $R'_3$ et $R_4$, identiques ou différents, désignent un radical alkyle en $C_1$-$C_4$, carboxyle, alcoxy($C_1$-$C_4$)carbonyle, monohydroxyalkyle en $C_1$-$C_4$, polyhydroxyalkyle en $C_2$-$C_4$, alcoxy($C_1$-$C_4$)alkyle en $C_1$-$C_4$, monoalkyl($C_1$-$C_4$)aminoalkyle en $C_1$-$C_4$ ou dialkyl($C_1$-$C_4$) aminoalkyle en $C_1$-$C_4$;

- $R_5$ désigne un atome d'hydrogène, un radical alkyle en $C_1$-$C_4$, monohydroxyalkyle en $C_1$-$C_4$, polyhydroxyalkyle en $C_2$-$C_4$, alcoxy($C_1$-$C_4$)alkyle en $C_1$-$C_4$, monoalkyl($C_1$-$C_4$)aminoalkyle en $C_1$-$C_4$ ou dialkyl($C_1$-$C_4$) aminoalkyle en $C_1$-$C_4$;

lesdits radicaux alkyle ou alcoxy pouvant être linéaires ou ramifiés.

Les sels d'addition avec un acide des composés de formule (I) ou (II) constituent également un objet de l'invention, et peuvent être notamment choisis parmi les chlorhydrates, les bromhydrates, les sulfates, les tartrates, les lactates ou les acétates.

Chacune des formules (I) ou (II) définies ci-dessus peut donner lieu à plusieurs formes tautomères dont la prépondérance et/ou la stabilité de chaque forme tautomère dépendra de la nature des différents substituants $R_1$, $R_2$, $R_3$, $R'_3$ et $R_4$.

La formule (I) peut donner lieu aux formes tautomères suivantes:

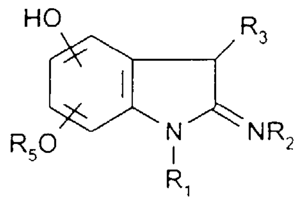

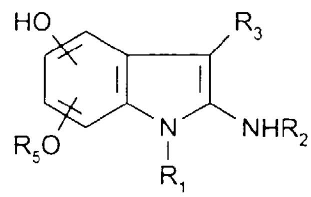

Parmi les composés de formule (I) ou (II) préférentiels et leurs sels d'addition avec un acide, on peut citer plus particulièrement le 5,6-dihydroxy-1,3-dihydro-indol-2-ylidèneamine et ses sels d'addition avec un acide.

Les composés particuliers de formule (IA) ou (IIA) suivantes ou leurs autres formes tautomères :

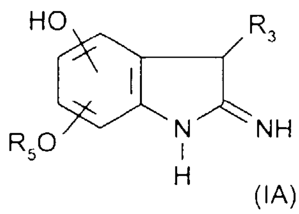

(IA)

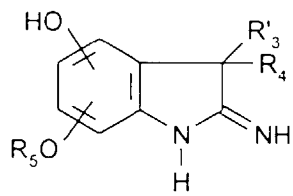

(II A)

dans lesquelles les radicaux $R_3$, $R'_3$, $R_4$ et $R_5$ ont les mêmes significations que celles indiquées ci-dessus dans les définitions des formules (I) et (II), peuvent être obtenus selon le procédé décrit dans le brevet RG Glushkov, USSR Patent 179 320 (1965) et dans le document Chem. Abstr 65, 2225(1966)) et correspondant aux schémas A et A' suivants:

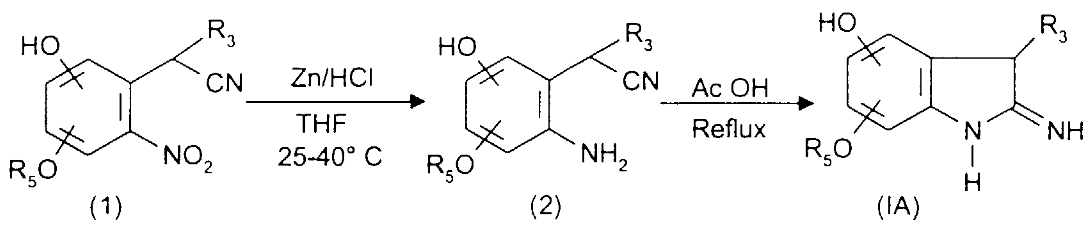


(1) (2) (IA)

**SCHEMA A**

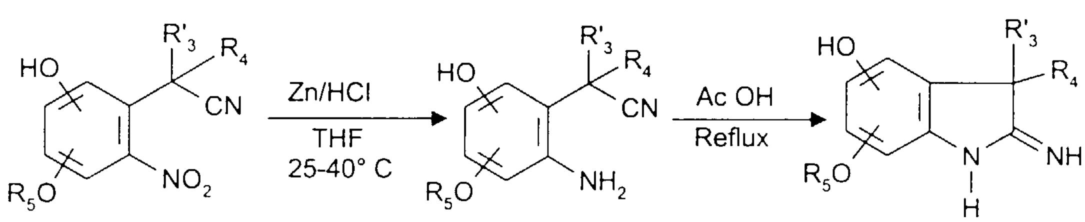


(1') (2') (II A)

**SCHEMA A'**

Dans les schémas A et A' définis ci-dessus, les significations des radicaux $R_3$, $R'_3$, $R_4$ et $R_5$ dans les formules (1), (1'), (2) et (2') sont identiques à celles indiquées précédemment dans la formule (I) ou (II).

Il s'agit d'un procédé en deux étapes à partir des composés de départ de formule (1) ou (1') ayant pour structure celle d'ortho-nitro phénylacétonitriles dont la méthode de synthèse est comme dans la littérature (M. MAKOSZA, J. WINIARSKI, Acc. Chem. Res., 87, 1987, 282 ; M. MAKOSZA, W. DANIKIEWICZ, K. WOJCIECHOWSKI; Liebigs, Ann. Chem. 1988, 203).

La première étape est soit une réduction chimique en présence d'un solvant organique à l'aide de métaux comme le zinc ou l'étain, soit une hydrogénation sélective à l'aide d'un catalyseur comme le palladium ou le platine. Les solvants utilisés sont de préférence des éthers et plus particulièrement le tétrahydrofurane (THF). La température de réaction est comprise de préférence entre 25° C et la température de reflux du solvant et plus particulièrement entre 25 et 40° C.

La seconde étape est une réaction de cyclisation en milieu acide en présence d'un solvant organique. On utilise de préférence l'acide acétique. La température de réaction est celle de reflux du solvant. Le produit final de formule (IA) ou (IIA) est isolé de préférence sous forme de sel d'addition d'acide. On l'obtient par précipitation du milieu réactionnel en milieu acide. Par exemple, pour obtenir un chlorhydrate, on fait passer un courant de HCl gazeux en fin de réaction.

Les dérivés de 2-imino-2,3-dihydro-1H-indoles de formule (I) ou (II) définis ci-dessus sont généralement mis en oeuvre à l'aide de compositions qui constituent un autre objet de l'invention.

Les compositions tinctoriales destinées à être utilisées pour la teinture des fibres kératiniques et en particulier les fibres kératiniques humaines telles que les cheveux, conformes à l'invention, sont caractérisées par le fait qu'elles contiennent dans un milieu approprié pour la teinture, au moins un composé répondant à la formule (I) ou (II) définies ci-dessus.

La quantité de composé de formule (i) ou (II) utilisée dans la composition, est comprise généralement dans des proportions de 0,01 à 8 % environ en poids par rapport au poids total de la composition et de préférence de 0,03 à 5 % environ en poids.

Ces compositions peuvent se présenter sous des formes diverses, notamment sous forme de lotions plus ou moins épaissies, de crèmes, de mousses et de gels, éventuellement conditionnées sous forme d'aérosols.

Elles peuvent également constituer un élément d'un agent de teinture à plusieurs composants disposé dans un dispositif à plusieurs compartiments ou kit de teinture.

Le milieu approprié pour la teinture est de préférence un milieu aqueux qui doit être cosmétiquement acceptable lorsque les compositions sont destinées à être utilisées pour la teinture des cheveux humains vivants. Ce milieu aqueux peut être constitué par de l'eau ou un mélange eau/solvant(s).

Le pH des compositions est généralement compris entre 3 et 12.

Les solvants peuvent être choisis parmi les solvants organiques et préférentiellement parmi l'alcool éthylique, l'alcool propylique ou isopropylique, l'alcool tertiobutylique, l'éthylèneglycol, les éthers monométhylique, monoéthylique et monobutylique de l'éthylèneglycol, l'acétate du monoéthyléther de l'éthyléneglycol, le propylèneglycol, les monométhyléthers du propylèneglycol et du dipropylèneglycol, le lactate de méthyle.

Les solvants particulièrement préférés sont l'alcool éthylique, le propylèneglycol et le monobutyléther d'éthylèneglycol.

Les composés conformes à l'invention présentent l'avantage de pouvoir être utilisés dans un milieu essentiellement aqueux.

Il est également possible d'utiliser un milieu constitué de solvants anhydres choisis parmi les solvants définis ci-dessus. La composition est dans ce cas soit mélangée au moment de l'emploi avec un milieu aqueux, soit appliquée sur les fibres kératiniques mouillées au préalable par une composition aqueuse.

On appelle, conformément à l'invention, un milieu solvant anhydre, un milieu contenant moins de 1 % d'eau.

Lorsque le milieu approprié pour la teinture est constitué par un mélange eau/solvant(s), les solvants sont utilisés dans des concentrations comprises entre 0,5 et 75 % en poids environ par rapport au poids total de la composition, et de préférence dans des proportions inférieures à 20 % en poids environ.

Les compositions conformes à l'invention peuvent contenir des adjuvants habituellement utilisés pour la teinture des fibres kératiniques et en particulier des adjuvants cosmétiquement acceptables lorsque ces compositions sont appliquées pour la teinture des cheveux humains vivants.

Ces compositions peuvent contenir notamment des amides gras dans des proportions préférentielles de 0,05 à 10 % en poids, des agents tensio-actifs anioniques, cationiques, non ioniques, amphotères ou leurs mélanges présents de préférence dans des proportions comprises entre 0,1 à 50 % en poids, des agents épaississants, des parfums, des agents séquestrants, des agents filmogènes, des agents de traitement, des agents dispersants, des agents de conditionnement, des agents conservateurs, des agents opacifiants, des agents de gonflement des fibres kératiniques.

Les agents épaississants sont choisis parmi l'alginate de sodium, la gomme arabique, la gomme de guar, les hétérobiopolysaccharides, tels que la gomme de xanthane, les scléroglucanes, les dérivés de cellulose, tels que la

méthylcellulose, l'hydroxyéthylcellulose, l'hydroxypropylcellulose, le sel de sodium de la carboxyméthylcellulose et les polymères d'acide acrylique réticulés de préférence.

On peut également utiliser les agents épaississants minéraux, tels que la bentonite.

Ces épaississants sont utilisés seuls ou en mélange et sont présents de préférence dans des proportions comprises entre 0,1 et 5 % en poids par rapport au poids total de la composition et avantageusement entre 0,5 et 3 % en poids.

Les agents alcalinisants utilisables dans les compositions peuvent être en particulier des amines, telles que les alcanolamines, des alkylamines, des hydroxydes ou carbonates alcalins ou d'ammonium.

Les agents d'acidification utilisables dans ces compositions peuvent être choisis parmi l'acide lactique, l'acide acétique, l'acide tartrique, l'acide phosphorique, l'acide chlorhydrique, l'acide citrique.

Il est bien entendu possible d'utiliser tout autre agent alcalinisant ou acidifiant acceptable, notamment dans le cas de la teinture des cheveux en cosmétique.

Lorsque les compositions sont utilisées sous forme de mousse, elles peuvent être conditionnées sous pression et dans un dispositif aérosol en présence d'un agent propulseur et d'au moins un générateur de mousse.

Les agents générateurs de mousse peuvent être des polymères moussants anioniques, cationiques, non ioniques, amphotères ou leurs mélanges ou des agents tensio-actifs du type de ceux définis ci-dessus.

Le procédé de teinture des fibres kératiniques, en particulier des fibres kératiniques humaines, qui constitue un autre objet de l'invention, est essentiellement caractérisé par le fait qu'il consiste à appliquer sur ces fibres une composition (A) définie ci-dessus, à maintenir la composition au contact des fibres pendant un temps suffisant pour développer la coloration, soit à l'air, soit à l'aide d'un système oxydant, à rincer et éventuellement à laver les fibres ainsi teintes.

Selon une première forme de mise en oeuvre de l'invention, la coloration des fibres peut être effectuée sans addition d'un agent oxydant extérieur, au seul contact de l'air.

Selon une autre forme de réalisation, la couleur est révélée à l'aide d'un système oxydant chimique choisi parmi:

(i) les ions iodure et du peroxyde d'hydrogène, la composition (A) contenant le composé de formule (I) ou (II) comprenant dans ce cas en plus, soit des ions iodure, soit du peroxyde d'hydrogène, et l'application de la composition (A) est précédée ou suivie par l'application d'une composition (B) qui contient dans un milieu approprié pour la teinture, soit :

(a) du peroxyde d'hydrogène à un pH compris entre 2 et 12 et de préférence entre 2 et 7, lorsque la composition (A) contient des ions iodure, soit :

(b) des ions iodure à un pH compris entre 3 et 11 lorsque la composition (A) contient du peroxyde d'hydrogène ;

(ii) les ions nitrite ; l'application de la composition (A) contenant le composé de formule (I) ou (II) étant suivie par l'application d'une composition aqueuse (B) présentant un pH acide, la composition (A) ou la composition (B) contenant au moins un nitrite ;

(iii) les oxydants choisis parmi le peroxyde d'hydrogène, l'acide périodique et ses sels hydrosolubles, l'hypochlorite de sodium, la chloramine T, la chloramine B, le ferricyanure de potassium, l'oxyde d'argent, le réactif de Fenton, l'oxyde de plomb (IV), le sulfate de césium, le persulfate d'ammonium, les chlorites alcalins ; ces oxydants étant présents dans la composition (A) contenant le composé de formule (I) ou (II) ou appliqués simultanément ou séquentiellement au moyen d'une composition (B) les contenant dans un milieu approprié pour la teinture ;

(iv) les anions d'un métal choisis parmi les permanganates ou bichromates, ces oxydants étant appliqués au moyen d'une composition (B) aqueuse, à un pH de 2 à 10, avant l'application de la composition (A) ;

(v) les sels métalliques des groupes 3 à 8 du tableau périodique, ces sels métalliques étant appliqués au moyen d'une composition (B) les contenant dans un milieu approprié pour la teinture, la composition (B) étant appliquée préalablement ou postérieurement à l'application de la composition (A) ;

(vi) les sels de terre rares, ces sels de terres rares étant appliqués au moyen d'une composition (B) les contenant dans un milieu approprié pour la teinture, la composition (B) étant appliquée préalablement ou postérieurement à l'application de la composition (A) contenant le composé de formule (I) ou (II) ;

(vii) un dérivé quinonique choisi parmi les ortho- ou parabenzoquinones, les ortho- ou parabenzoquinones monoimines ou diimines, les 1,2- ou 1,4-naphtoquinones, les ortho- ou parabenzoquinones sulfonimides, les α,ω-alkylène-bis-1,4-benzoquinones ou les 1,2- ou 1,4-naphtoquinones monoimines ou diimines au moyen d'une composition (B) les contenant dans un milieu approprié pour la teinture, le composé de formule (I) ou (II) et les dérivés quinoniques étant choisis de façon à ce que la différence de potentiel d'oxydoréduction ΔE entre le potentiel d'oxydoréduction Ei du composé de formule (I) ou (II) déterminé à pH 7 en milieu phosphate sur électrode de carbone vitreux par voltamétrie et le potentiel d'oxydoréduction Eq du dérivé quinonique déterminé à pH 7 en milieu phosphate par polarographie sur électrode de mercure par rapport à l'électrode au calomel saturé, soit telle que :

$$\Delta E = Ei - Eq \leq 320 \text{ millivolts ;}$$

la composition (B) étant appliquée préalablement ou postérieurement à l'application de la composition (A) contenant le composé de formule (I) ou (II).

Selon une forme préférée de l'invention, l'application des compositions (A) et (B) est séparée par une étape de rinçage à l'eau.

A titre de dérivés quinoniques utilisables dans ce procédé, on peut citer :

- la 1,4-benzoquinone,
- la 2-méthoxy 1,4-benzoquinone,
- la 2-méthyl 1,4-benzoquinone,
- la 2,6-diméthyl 1,4-benzoquinone,
- la 2,3,5-trichloro 6-méthyl 1,4-benzoquinone,
- la 2-acétylamino 1,4-benzoquinone,
- la 2-acétylamino 3,5-diméthyl 1,4-benzoquinone,
- la 2,6-diméthyl 5-acétylamino 1,4-benzoquinone,
- la 2-chloro 1,4-benzoquinone,
- la tétrachloro 1,2-benzoquinone,
- la 2,3-diméthoxy 1,4-benzoquinone,
- la 2-β-carboxyéthoxy 1,4-benzoquinone,
- la 2-méthoxyméthyl 1,4-benzoquinone,
- la 2-β-hydroxyéthyl 1,4-benzoquinone,
- la 2-β-hydroxyéthylthio 1,4-benzoquinone,
- la 2,5-bis-β-hydroxyéthylthio 1,4-benzoquinone,
- la 2-β,γ-dihydroxypropylthio 1,4-benzoquinone,
- la 2-β-carboxyéthylthio 1,4-benzoquinone,
- la 2-carboxyméthyl 1,4-benzoquinone,
- la 2-β-hydroxyéthylthio 6-méthyl 1,4-benzoquinone,
- la 2-méthoxycarbonyl 3-méthoxy 1,4-benzoquinone,
- la 2-méthoxycarbonyl 1,4-benzoquinone,
- la 2-méthylthio 1,4-benzoquinone,
- la 2-diméthylamino 1,4-benzoquinone,
- la 2-acétylamino 5-méthoxy 1,4-benzoquinone,
- la 2-(β-hydroxyéthylthio)méthyl 1,4-benzoquinone,
- la 2-(méthylthio)méthyl 1,4-benzoquinone,
- la 4,5-diméthoxy 1,2-benzoquinone,
- la 4-méthyl 5-chloro 1,2-benzoquinone,
- la 4,5-diméthyl 1,2-benzoquinone,
- la 2,3-diméthyl 1,4-benzoquinone,
- la 2-β-hydroxyéthoxy 1,4-benzoquinone,
- la N-méthyl sulfonyl 1,4-benzoquinone monoimine,
- la N-phényl sulfonyl 1,4-benzoquinone monoimine,
- la 1,4-naphtoquinone,
- la 1,2-naphtoquinone,
- l'acide 1,2-naphtoquinone 4-sulfonique,
- la 2,3-dichloro 1,4-naphtoquinone, et
- la N-2,6-dichloro 1,4-benzoquinone imine.

Selon une première variante du procédé de teinture mettant en oeuvre des systèmes oxydants, on applique sur les matières kératiniques une composition (A) contenant dans un milieu approprié pour la teinture, au moins un composé de formule (I) ou (II) en association avec des ions iodure, l'application de la composition (A) étant précédée ou suivie par l'application d'une composition (B) qui contient dans un milieu approprié pour la teinture, du peroxyde d'hydrogène.

Ce procédé peut également être mis en oeuvre en appliquant sur les fibres kératiniques au moins une composition (A) contenant dans un milieu approprié pour la teinture, le composé de formule (I) ou (II) en association avec du peroxyde d'hydrogène, ayant un pH compris entre 2 et 7 et de préférence entre 3,5 et 7, l'application de la composition (A) étant précédée ou suivie par l'application d'une composition (B) qui contient dans un milieu approprié pour la teinture, des ions iodure.

L'ion iodure dans cette variante du procédé, est choisi de préférence parmi les iodures de métaux alcalins, alcalino-terreux ou d'ammonium. L'iodure est plus particulièrement constitué par l'iodure de potassium.

Les ions iodure sont présents dans les compositions (A) ou (B) dans des proportions comprises généralement entre 0,007 et 4 % en poids exprimées en ions I et de préférence entre 0,08 et 1,5 % en poids par rapport au poids total de la composition (A) ou (B).

Selon une seconde variante, ce procédé peut être mis en oeuvre en utilisant comme agent oxydant pour révéler la coloration, un nitrite. Les nitrites plus particulièrement utilisables conformément à l'invention, sont :

- les nitrites de métaux alcalins, alcalino-terreux ou d'ammonium ou de tout autre cation cosmétiquement acceptable lorsqu'il est utilisé pour la teinture des cheveux humains vivants ;

- les dérivés organiques des nitrites tels que par exemple le nitrite d'amyle ;

- les vecteurs de nitrites, c'est-à-dire des composés qui par transformation forment des nitrites du type défini ci-dessus.

Les nitrites particulièrement préférés sont les nitrites de sodium, de potassium ou d'ammonium.

Cette variante du procédé est mise en oeuvre en appliquant sur les matières kératiniques, la composition (A) à base du composé de formule (I) ou (II) définies ci-dessus, puis d'une composition aqueuse acide (B), la composition (A) ou (B) contenant au moins un nitrite.

Les nitrites sont généralement utilisés dans des proportions comprises entre 0,02 et 1 mole/litre.

Selon une troisième variante de ce procédé, les oxydants sont choisis parmi le peroxyde d'hydrogène, la chloramine B, l'acide périodique et ses sels hydrosolubles, l'hypochlorite de sodium, le ferricyanure de potassium, l'oxyde d'argent, le réactif de Fenton, l'oxyde de plomb (IV), le sulfate de césium, le persulfate d'ammonium. Ces agents sont de préférence appliqués sur les fibres au moyen d'une composition (B) et après l'application de la composition (A).

Ces agents oxydants sont présents dans des proportions suffisantes pour développer une coloration et de préférence dans des proportions comprises entre 0,004 mole et 0,04 mole pour 100 g de composition.

Selon une quatrième variante de ce procédé, on applique dans un premier temps sur les fibres kératiniques, une composition (B) contenant dans un milieu approprié pour la teinture, à un pH compris entre 2 et 10, un anion d'un métal ayant une bonne affinité pour la kératine et ayant un potentiel d'oxydoréduction supérieur à celui des composés de formule (I) ou (II). Cet anion est choisi de préférence parmi les permanganates ou les bichromates et plus particulièrement le permanganate de potassium et le bichromate de sodium.

Ces anions métalliques sont généralement utilisés à des molalités en anions supérieures à $10^{-3}$ mole/1000 g jusqu'à de préférence de 1 mole/1000 g.

Dans un second temps, on applique une composition (A) contenant dans un milieu approprié pour la teinture, à un pH compris entre 4 et 10, au moins un composé répondant à la formule (I) ou (II) définie ci-dessus.

Les compositions contenant les anions ne doivent pas contenir d'agents organiques ayant un effet réducteur sur les anions.

Selon une cinquième variante de l'invention, on met en oeuvre des catalyseurs d'oxydation choisis parmi les sels métalliques tels que les sels de manganèse, le chlorure de cobalt, le chlorure ferrique, le chlorure cuivrique, le nitrate d'argent ammoniacal.

Les sels préférés sont les sels de cuivre. Ces sels sont utilisés dans des proportions de 0,01 à 2 % exprimées en ions métalliques par rapport au poids total de la composition mise en oeuvre et contenant ces sels.

Selon cette variante, on met les fibres kératiniques, en particulier les cheveux, en contact avec une composition (B) contenant dans un milieu approprié pour la teinture, le sel métallique, avant ou après l'application de la composition (A) contenant le composé de formule (I) ou (II) et on rince de préférence entre les deux étapes.

La forme de réalisation préférée consiste à appliquer un sel cuivrique dans un premier temps et la composition (A) contenant le composé de formule (I) ou (II) , dans un deuxième temps.

On peut faire suivre cette teinture après rinçage, de l'application d'une solution de peroxyde d'hydrogène pour éventuellement éclaircir la couleur obtenue.

Selon une sixième variante, on utilise des sels de terres rares. Les sels de terres utilisables conformément à l'invention sont choisis parmi les sels de Lanthanides, et notamment les sels de Cérium $Ce^{3+}$, $Ce^{4+}$, de Lanthane $La^{3+}$, d'Europium $Eu^{2+}$, $Eu^{3+}$, de Gadolinium $Gd^{3+}$, d'Ytterbium $Yb^{2+}$, $Yb^{3+}$, de Dysprosium $Dy^{3+}$. Les sels préférés sont en particulier les sulfates, chlorures ou nitrates.

Ces sels de terres rares sont présents dans des proportions comprises entre 0,1 et 8 % en poids par rapport au poids total de la composition.

On utilise de préférence les sels de Cérium $Ce^{3+}$ et $Ce^{4+}$ sous forme de sulfates et de chlorures.

Selon une septième variante, la composition contenant le dérivé quinonique est appliquée avant ou après la composition (A) contenant le composé de formule (I) ou (II).

A titre de dérivé quinonique préféré, on peut citer la 1,4-benzoquinone et la 2-β-hydroxyéthylthio 1,4-benzoquinone.

La concentration en dérivés quinoniques est de préférence comprise entre 0,005 et 1 mole/litre dans la composition (B). Le pH de la composition (B) est compris entre 2 et 10 et de préférence inférieur à 7.

Lorsqu'on met en oeuvre des compositions à base de peroxyde d'hydrogène dans les différents procédés décrits ci-dessus, la teneur en peroxyde d'hydrogène est généralement comprise entre 1 et 40 volumes et de préférence entre 2 et 10 volumes et plus particulièrement entre 3 et 10 volumes.

L'invention a également pour objet un agent de coloration des fibres kératiniques et en particulier de fibres kératiniques humaines, à plusieurs composants, destiné notamment à être utilisé dans la mise en oeuvre du procédé de teinture défini ci-dessus et utilisant un système oxydant. Dans ce cas, l'agent de teinture comprend au moins deux composants dont un premier est constitué par la composition (A) définie ci-dessus et contenant le composé de formule (I) ou (II) et l'autre composant est constitué par l'une des compositions (B) également définie ci-dessus.

Les composants (A) et (B) respectifs sont choisis selon les différentes variantes du procédé exposées ci-dessus.

L'invention a également pour objet un dispositif à plusieurs compartiments ou encore "kit de teinture" ou "nécessaire de teinture", comportant tous les composants destinés à être appliqués dans une même teinture sur les fibres kératiniques en application unique ou successives avec ou sans pré-mélange comme mentionné ci-dessus.

De tels dispositifs sont connus en eux-mêmes et peuvent comporter un premier compartiment contenant la composition (A) contenant le composé de formule (I) ou (II) dans un milieu approprié pour la teinture, et dans un second compartiment une composition (B) du type défini ci-dessus et contenant l'agent oxydant.

Les dispositifs à plusieurs compartiments utilisables conformément à l'invention, peuvent être équipés des moyens de mélanges au moment de l'emploi et leur contenu peut être conditionné sous atmosphère inerte.

Lorsque le milieu contenant le composé de formule (I) ou (II) est anhydre, on peut prévoir un troisième compartiment contenant un milieu aqueux approprié pour la teinture et destiné à être mélangé tout juste avant l'emploi avec la composition du premier compartiment.

Le composé de formule (I) ou (II), les compositions et le procédé conformes à l'invention, peuvent être mis en oeuvre pour teindre des cheveux naturels ou déjà teints, permanentés ou non, défrisés ou non, ou des cheveux fortement ou légèrement décolorés et éventuellement permanentés.

Il est également possible de les utiliser pour la teinture de la fourrure ou de la laine.

Les exemples qui suivent sont destinés à illustrer l'invention sans pour autant présenter un caractère limitatif.

## EXEMPLES

**EXEMPLE n°1 : Préparation du chlorhydrate de 5,6-dihydroxy-1,3-dihydro-indol-2-ylidèneamine**

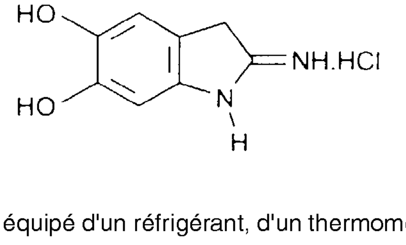

Dans un ballon tricol de 250 cc équipé d'un réfrigérant, d'un thermomètre et d'une ampoule à introduction de solide, on a solubilisé 15,3 g de (4,5-dihydroxy-2-nitro-phényl)acétonitrile dans un mélange THF/HCl à 37 % = 70 cc/54 cc. On a ensuite ajouté 35 g de zinc en poudre par portions en maintenant la température au dessous de 60°C. Après complète addition du zinc, on a laissé revenir le milieu réactionnel à température ambiante, puis on a filtré le milieu sur célite. On a saturé le filtrat porté à la température d'un bain de glace avec de l'acide chlorhydrique gazeux. Le chlorhydrate de 5,6-dihydroxy-1,3-dihydro-indol-2-ylideneamine a précipité. On l'a filtré, lavé à l'éther de pétrole et séché sous vide sur anhydride phosphorique et potasse à 40°C. (Rendement = 90 %).
RMN $^1H$ et $^{13}C$ conformes à la structure.

| RMN $^1H$ (DMSO d6; 400 mhz) | | |
|---|---|---|
| **δ (ppm)** | **multiplicité** | **intégration** |
| 3.97 | s | 2 |
| 6.67 | s | 1 |
| 6.80 | s | 1 |
| 8.00-9.00 | m | 2 |
| 9.42-9.66 | 2 s | 2 |
| 11.69 | s | 1 |

## EXEMPLES DE FORMULATION ET DE PROCEDES DE TEINTURE

### EXEMPLE 1

On a préparé la composition tinctoriale A suivante (teneurs en grammes) :

- 5,6-dihydroxy-1,3-dihydro-indol-2-ylidèneamine, HCl 1 g
- Ammoniaque à 20% 2 g
- Eau déminéralisée q.s.p. 100 g

PROCEDE DE TEINTURE 1 (oxydation par l'oxygène de l'air)

La composition A a été appliquée pendant 30 minutes sur des mèches de cheveux gris naturels à 90 % de blancs, permanentés ou non, à raison de 28 g pour 3 g de cheveux. Après rinçage, lavage avec un shampooing standard et séchage, les mèches ont été teintes dans les nuances figurant dans le tableau I ci-dessous :

PROCEDE DE TEINTURE 2 (oxydation par de l'eau oxygénée)

Au moment de l'emploi, La composition A a été mélangée avec un poids égal d'eau oxygénée à 20 volumes (6% en poids).
Le mélange obtenu a été appliqué pendant 30 minutes, sur des mèches de cheveux gris naturels à 90 % de blancs, permanentés ou non, à raison de 28 g pour 3 g de cheveux. Après rinçage, lavage avec un shampooing standard et séchage, les mèches ont été teintes dans les nuances figurant dans le tableau I ci-dessous :

Tableau I

| Procédé de teinture | 1 | 2 |
|---|---|---|
| **Nuance obtenue sur cheveux gris naturels à 90% de blancs** | Blond foncé gris mat puissant | Blond très clair beige |
| **Nuance obtenue sur cheveux gris à 90% de blancs permanentés** | Châtain gris foncé mat | Blond foncé doré mat |

On obtient une coloration puissante et ayant une bonne résistance à l'action de la lumière.

## Revendications

1. Dérivés de 2-imino-2,3-dihydro-1H-indoles répondant à la formule (I) ou (II) suivantes:

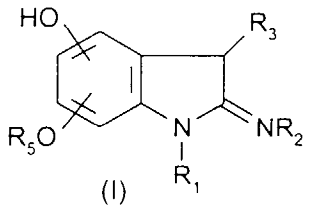

(I)

ou

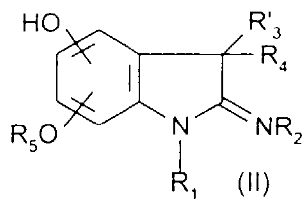

(II)

dans lesquelles:

- $R_1$, $R_2$ et $R_3$, identiques ou différents, désignent un atome d'hydrogène, un radical alkyle en $C_1$-$C_4$, carboxyle, alcoxy($C_1$-$C_4$)carbonyle, monohydroxyalkyle en $C_1$-$C_4$, polyhydroxyalkyle en $C_2$-$C_4$, alcoxy($C_1$-$C_4$)alkyle en $C_1$-$C_4$, monoalkyl($C_1$-$C_4$)aminoalkyle en $C_1$-$C_4$ ou dialkyl($C_1$-$C_4$)aminoalkyle en $C_1$-$C_4$ ;
- $R'_3$ et $R_4$, identiques ou différents, désignent un radical alkyle en $C_1$-$C_4$, carboxyle, alcoxy($C_1$-$C_4$)carbonyle, monohydroxyalkyle en $C_1$-$C_4$, polyhydroxyalkyle en $C_2$-$C_4$, alcoxy($C_1$-$C_4$)alkyle en $C_1$-$C_4$, monoalkyl($C_1$-$C_4$) aminoalkyle en $C_1$-$C_4$ ou dialkyl($C_1$-$C_4$) aminoalkyle en $C_1$-$C_4$ ;
- $R_5$ désigne un atome d'hydrogène, un radical alkyle en $C_1$-$C_4$, monohydroxyalkyle en $C_1$-$C_4$, polyhydroxyalkyle en $C_2$-$C_4$, alcoxy($C_1$-$C_4$)alkyle en $C_1$-$C_4$, monoalkyl($C_1$-$C_4$)aminoalkyle en $C_1$-$C_4$ ou dialkyl($C_1$-$C_4$) aminoalkyle en $C_1$-$C_4$ ; lesdits radicaux alkyle ou alcoxy pouvant être linéaires ou ramifiés ; ainsi que leurs sels d'addition avec un acide ;

et leurs possibles formes tautomères.

**2.** Dérivés selon la revendication 1, caractérisés par le fait que les sels d'addition avec un acide sont choisis dans le groupe constitué par les chlorhydrates, les bromhydrates, les sulfates, les tartrates, les acétates, les lactates.

**3.** 5,6-dihydroxy-1,3-dihydro-indol-2-ylidèneamine et sels d'addition avec un acide.

**4.** Procédé de préparation des composés de formule (IA) ou (IIA) suivantes ou de l'une de leurs formes tautomères :

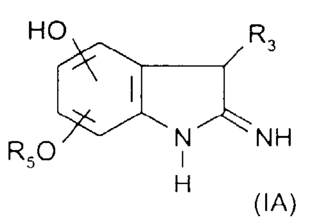

(IA)

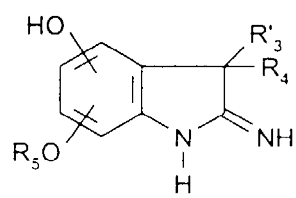

(II A)

dans lesquelles les radicaux $R_3$, $R'_3$, $R_4$ et $R_5$ ont les mêmes significations que celles indiquées dans la revendication 1, caractérisé par le fait que l'on fait réagir un composé de formule (1) ou (1') suivantes :

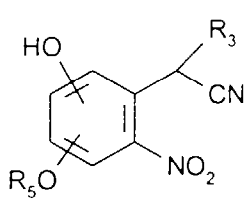

(1)

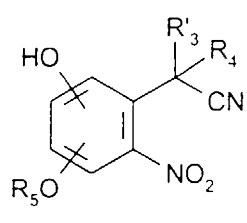

(1')

dans lesquelles les significations des radicaux $R_3$, $R'_3$, $R_4$ et $R_5$ sont identiques à celles indiquées pour la formule (I) ou (II) de la revendication 1, en effectuant soit une réduction chimique en présence d'un solvant organique à

l'aide de métaux, soit une réaction d'hydrogénation sélective à l'aide d'un catalyseur pour obtenir un composé de formule (2) ou (2') suivantes:

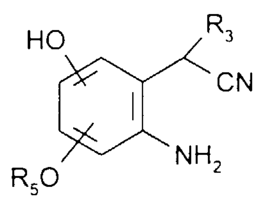

(2)

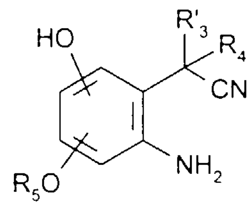

(2')

dans lesquelles les radicaux $R_3$, $R'_3$, $R_4$ et $R_5$ ont les mêmes significations que celles indiquées dans la revendication 1 ;
puis que l'on effectue une réaction de cyclisation en milieu acide en présence d'un solvant organique.

**5.** Composition tinctoriale destinée à être utilisée pour la teinture des fibres kératiniques, caractérisée par le fait qu'elle contient dans un milieu approprié pour la teinture au moins un composé de formule (I) ou (II) ou au moins l'un de leurs sels d'addition avec un acide tels que définis selon l'une quelconque des revendications 1 à 3 ; ladite composition étant exempte de base d'oxydation.

**6.** Composition selon la revendication 5, caractérisée par le fait que le composé de formule (I) ou (II) est présent dans la composition dans des proportions comprises entre 0,01 à 8 % en poids par rapport au poids total de la composition.

**7.** Composition selon la revendication 6, caractérisée par le fait que le composé de formule (I) ou (II) est présent dans la composition dans des proportions comprises entre 0,03 et 5 % en poids par rapport au poids total de la composition.

**8.** Composition selon l'une quelconque des revendications 5 à 7, caractérisée par le fait que le milieu approprié pour la teinture est un milieu aqueux constitué par de l'eau ou un mélange eau/solvant(s).

**9.** Composition selon la revendication 8, caractérisée par le fait que les solvants sont choisis parmi l'alcool éthylique, l'alcool propylique ou isopropylique, l'alcool tertiobutylique, l'éthylèneglycol, les éthers monométhylique, monoéthylique et monobutylique de l'éthylèneglycol, l'acétate du monoéthyléther de l'éthylèneglycol, le propylèneglycol, les monométhyléthers du propylèneglycol et du dipropylèneglycol, le lactate de méthyle.

**10.** Composition selon l'une quelconque des revendications 5 à 9, caractérisée par le fait qu'elle contient au moins un adjuvant choisi parmi les amides gras, les agents tensioactifs anioniques, cationiques, non-ioniques, amphotères ou leurs mélanges, les agents épaississants, les parfums, les agents séquestrants, les agents opacifiants, les agents de gonflement des fibres kératiniques et leurs mélanges.

**11.** Composition selon l'une quelconque des revendications 5 à 10, caractérisée par le fait qu'elle présente un pH compris entre 3 et 12.

**12.** Procédé de teinture des fibres kératiniques caractérisé par le fait que l'on applique sur ces fibres au moins une composition telle que définie dans l'une quelconque des revendications 5 à 11, que l'on maintient cette composition au contact des fibres pendant un temps suffisant pour développer une coloration, soit à l'air, soit à l'aide d'un système oxydant et qu'on rince ensuite.

**13.** Procédé selon la revendication 12, caractérisé par le fait que l'on laisse la coloration se développer au contact de l'air sans additionner d'agent oxydant extérieur.

**14.** Procédé selon la revendication 12, caractérisé par le fait que l'on applique sur les fibres une composition (A) contenant dans un milieu approprié pour la teinture, au moins un composé de formule (I) ou (II) tel que défini dans l'une quelconque des revendications 1 à 3, la couleur étant révélée à l'aide d'un système oxydant chimique constitué par :

(i) des ions iodure et du peroxyde d'hydrogène, la composition (A) contenant le composé de formule (I) ou (II) comprenant dans ce cas en plus, soit des ions iodure, soit du peroxyde d'hydrogène, et l'application de la composition (A) étant précédée ou suivie par l'application d'une composition (B) qui contient dans un milieu approprié pour la teinture, soit :

(a) du peroxyde d'hydrogène à un pH compris entre 2 et 12, lorsque la composition (A) contient des ions iodure, soit :

(b) des ions iodure à un pH compris entre 3 et 11 lorsque la composition (A) contient du peroxyde d'hydrogène ;

(ii) des ions nitrite ; l'application de la composition (A) contenant le composé de formule (I) ou (II) étant suivie par l'application d'une composition aqueuse (B) présentant un pH acide, la composition (A) ou la composition (B) contenant au moins un nitrite ;

(iii) des oxydants choisis parmi le peroxyde d'hydrogène, l'acide périodique et ses sels hydrosolubles, l'hypochlorite de sodium, la chloramine T, la chloramine B, le ferricyanure de potassium, l'oxyde d'argent, le réactif de Fenton, l'oxyde de plomb (IV), le sulfate de césium, le persulfate d'ammonium, les chlorites alcalins ; ces oxydants étant présents dans la composition (A) contenant le composé de formule (I) ou (II) ou appliqués simultanément ou séquentiellement au moyen d'une composition (B) les contenant dans un milieu approprié pour la teinture ;

(iv) des anions d'un métal choisis parmi les permanganates ou bichromates, ces oxydants étant appliqués au moyen d'une composition (B) aqueuse, à un pH de 2 à 10, avant l'application de la composition (A) ;

(v) des sels métalliques des groupes 3 à 8 du tableau périodique, ces sels métalliques étant appliqués au moyen d'une composition (B) les contenant dans un milieu approprié pour la teinture, la composition (B) étant appliquée préalablement ou postérieurement à l'application de la composition (A) ;

(vi) des sels de terres rares, ces sels de terres rares étant appliqués au moyen d'une composition (B) les contenant dans un milieu approprié pour la teinture, la composition (B) étant appliquée préalablement ou postérieurement à l'application de la composition (A) contenant le composé de formule (I) ou (II) ;

(vii) un dérivé quinonique choisi parmi les ortho- ou parabenzoquinones, les ortho- ou parabenzoquinones monoimines ou diimines, les 1,2- ou 1,4-naphtoquinones, les ortho- ou parabenzoquinones sulfonimides, les $\alpha$, $\omega$-alkylène-bis-1,4-benzoquinones ou les 1,2- ou 1,4-naphtoquinones monoimines ou diimines au moyen d'une composition (B) les contenant dans un milieu approprié pour la teinture, le composé de formule (I) et les dérivés quinoniques étant choisis de façon à ce que la différence de potentiel d'oxydoréduction $\Delta E$ entre le potentiel d'oxydoréduction Ei du composé de formule (I) ou (II) déterminé à pH 7 en milieu phosphate sur électrode de carbone vitreux par voltamétrie et le potentiel d'oxydoréduction Eq du dérivé quinonique déterminé à pH 7 en milieu phosphate par polarographie sur électrode de mercure par rapport à l'électrode au calomel saturé, soit telle que :

$$\Delta E = Ei - Eq \leq 320 \text{ millivolts ;}$$

la composition (B) étant appliquée préalablement ou postérieurement à l'application de la composition (A) contenant le composé de formule (I) ou (II).

**15.** Procédé selon la revendication 14, caractérisé par le fait que l'on applique sur les fibres kératiniques, au moins une composition (A) contenant dans un milieu approprié pour la teinture, un composé de formule (I) ou (II) en association avec du peroxyde d'hydrogène et ayant un pH compris entre 2 et 7, l'application de la composition (A) étant précédée ou suivie par l'application d'une composition (B) qui contient dans un milieu approprié pour la teinture, des ions iodure.

**16.** Procédé selon la revendication 14 ou 15, caractérisé par le fait que les ions iodure sont présents dans la composition (A) ou (B) dans des proportions comprises entre 0,007 et 4 % en poids exprimées en ions $I^-$ par rapport au poids total de la composition (A) ou (B).

**17.** Procédé selon la revendication 14, caractérisé par le fait que l'on applique sur les matières kératiniques une composition (A) contenant le composé de formule (I) ou (II) et que l'on applique ensuite une composition aqueuse acide (B), la composition (A) ou la composition (B) contenant au moins un nitrite choisi parmi les nitrites de métaux alcalins, alcalino-terreux ou d'ammonium ou de tout autre cation cosmétiquement acceptable, un dérivé organique de nitrite et des vecteurs de nitrite générant un nitrite du type défini ci-dessus, ces nitrites étant présents dans des proportions comprises entre 0,02 et 1 mole/litre.

**18.** Procédé selon la revendication 14, caractérisé par le fait que l'on applique sur les fibres kératiniques une composition (B) contenant dans un milieu approprié pour la teinture, à un pH compris entre 2 et 10, un anion d'un métal ayant une bonne affinité pour la kératine choisi parmi les permanganates ou les bichromates et que dans un second temps, on applique une composition (A) contenant dans un milieu approprié pour la teinture, à un pH compris entre 4 et 10, un composé de formules (I) ou (II) telles que définies à la revendication 1 ; ces permanganates ou bichromates étant utilisés dans des molalités en anions supérieures à $10^{-3}$ mole/1000 g et que les compositions ne contiennent pas d'agent organique ayant un effet réducteur sur les anions.

**19.** Procédé selon la revendication 14, caractérisé par le fait que l'on applique sur les fibres kératiniques une composition (A) contenant dans un milieu approprié pour la teinture, un composé de formules (I) ou (II) telles que définies à la revendication 1 et que l'on applique avant ou après la composition (A) une composition (B) contenant dans un milieu approprié pour la teinture, un sel métallique choisi parmi les sels de manganèse, de cobalt, de fer, de cuivre et d'argent, ces sels de métaux étant utilisés dans des proportions comprises entre 0,01 et 2 % en poids exprimé en ions métalliques par rapport au poids total de la composition.

**20.** Procédé selon la revendication 14, caractérisé par le fait que l'on applique une composition (A) contenant dans un milieu approprié pour la teinture, au moins un composé de formules (I) ou (II) telles que définies à la revendication 1 et qu'avant ou après cette composition, on applique une composition (B) contenant dans un milieu approprié pour la teinture, un sel de terres rares choisi parmi les sels de Cérium, de Lanthane, d'Europium, de Gadolinium, d'Ytterbium, de Dysprosium, ces sels de terres rares étant présents dans des proportions comprises entre 0,1 et 8 % en poids par rapport au poids total de la composition.

**21.** Procédé selon la revendication 14, caractérisé par le fait que l'on applique une composition (A) contenant dans un milieu approprié pour la teinture, au moins un composé de formules (I) ou (II) telles définies à la revendication 1, et qu'avant ou après cette composition, on applique une composition (B) contenant dans un milieu approprié un dérivé quinonique choisi parmi la 1,4-benzoquinone et la 2-β-hydroxyéthylthio 1,4-benzoquinone, ce dérivé quinonique étant présent dans des proportions comprises entre 0,005 et 1 mole/litre.

**22.** Procédé selon les revendications 14 à 16, caractérisé par le fait que lorsqu'on utilise comme moyen oxydant une composition à base de peroxyde d'hydrogène, la teneur en peroxyde d'hydrogène dans la composition est comprise entre 1 et 40 volumes.

**23.** Agent de teinture des fibres kératiniques, en particulier des fibres kératiniques humaines, à plusieurs composants, caractérisé par le fait qu'il comprend un premier composant constitué par une composition (A) telle que définie dans l'une quelconque des revendications 14 à 22 contenant un composé de formules (I) ou (II) telles que définies à la revendication 1, et un second composant constitué par l'une des compositions (B) définies dans l'une quelconque des revendications 14 à 22.

**24.** Dispositif à plusieurs compartiments ou "kit de teinture", caractérisé par le fait qu'il comporte un premier compartiment contenant une composition (A) telle que définie dans l'une quelconque des revendications 14 à 22 contenant un composé de formules (I) ou (II) telles que définies à la revendication 1, et un second compartiment contenant une composition (B) telle définie dans l'une quelconque des revendications 14 à 22.

## Patentansprüche

**1.** 2-Imino-2,3-dihydro-1H-indol-Derivate der folgenden Formel (I) oder (II) sowie die Additionssalze dieser Verbindungen mit einer Säure und ihre möglichen tautomeren Formen:

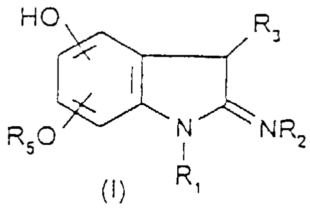

(I) oder

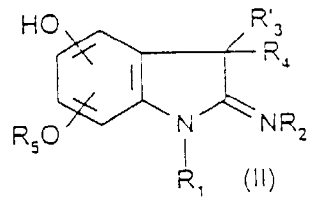

(II) ,

worin bedeuten:

- $R_1$, $R_2$ und $R_3$, die identisch oder voneinander verschieden sind, Wasserstoff, $C_{1-4}$-Alkyl, Carboxy, $C_{1-4}$-Alkoxycarbonyl, $C_{1-4}$-Monohydroxyalkyl, $C_{2-4}$-Polyhydroxyalkyl, $C_{1-4}$-Alkoxy-$C_{1-4}$-alkyl, $C_{1-4}$-Monoalkyl-$C_{1-4}$-aminoalkyl oder $C_{1-4}$-Dialkyl-$C_{1-4}$-aminoalkyl;
- $R_3$' und $R_4$, die identisch oder voneinander verschieden sind, $C_{1-4}$-Alkyl, Carboxy, $C_{1-4}$-Alkoxycarbonyl, $C_{1-4}$-Monohydroxyalkyl, $C_{2-4}$-Polyhydroxyalkyl, $C_{1-4}$-Alkoxy-$C_{1-4}$-alkyl, $C_{1-4}$-Monoalkyl-$C_{1-4}$-aminoalkyl oder $C_{1-4}$-Dialkyl-$C_{1-4}$-aminoalkyl;
- $R_5$ Wasserstoff, $C_{1-4}$-Alkyl, $C_{1-4}$-Monohydroxyalkyl, $C_{2-4}$-Polyhydroxyalkyl, $C_{1-4}$-Alkoxy-$C_{1-4}$-alkyl, $C_{1-4}$-Monoalkyl-$C_{1-4}$-aminoalkyl oder $C_{1-4}$-Dialkyl-$C_{1-4}$-aminoalkyl,

wobei die Alkyl- oder Alkoxygruppen geradkettig oder verzweigt vorliegen können.

**2.** Derivate nach Anspruch 1, dadurch gekennzeichnet, daß die Additionssalze mit einer Säure unter den Hydrochloriden, Hydrobromiden, Sulfaten, Tartraten, Acetaten oder Lactaten ausgewählt sind.

**3.** 5,6-Dihydroxy-1,3-dihydro-indol-2-ylidenamin und seine Additionssalze mit einer Säure.

**4.** Verfahren zur Herstellung von Verbindungen der folgenden Formeln (IA) oder (IIA) oder einer ihrer tautomeren Formen:

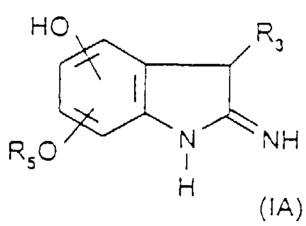

(IA)

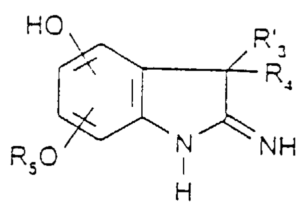

(II A) ,

worin die Gruppen $R_3$, $R_3$', $R_4$ und $R_5$ die in Anspruch 1 angegebenen Bedeutungen aufweisen,
dadurch gekennzeichnet, daß
eine Verbindung der folgenden Formeln (1) oder (1') umgesetzt wird:

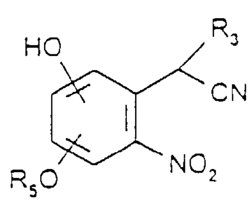

(1)

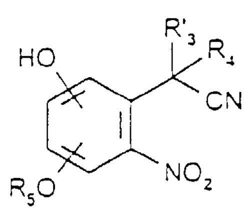

(1') ,

worin die Gruppen $R_3$, $R_3$', $R_4$ und $R_5$ die für Formel (I) oder (II) in Anspruch 1 angegebenen Bedeutungen aufweisen, wobei entweder eine chemischen Reduktion unter Verwendung eines Metalls in Gegenwart eines organischen Lösungsmittels oder eine selektive Hydrierung unter Verwendung eines Katalysators durchgeführt wird, um eine Verbindung der folgenden Formeln (2) oder (2') herzustellen:

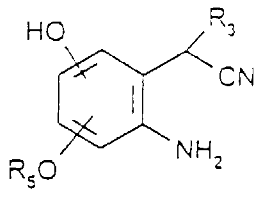

(2)

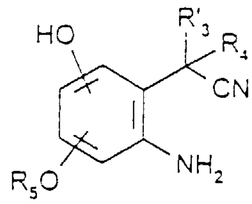

(2')

,

worin die Gruppen $R_3$, $R_3$', $R_4$ und $R_5$ die in Anspruch 1 angegebenen Bedeutungen aufweisen,
und anschließend eine Cyclisierung in einem sauren Medium in Gegenwart eines organischen Lösungsmittels durchgeführt wird.

**5.** Zusammensetzung zum Färben, die zum Färben von Keratinfasern verwendet werden soll, dadurch gekennzeichnet, daß sie in einem zum Färben geeigneten Medium mindestens eine Verbindung der Formel (I) oder (II) oder mindestens ein Additionssalz dieser Verbindungen mit einer Säure nach einem der Ansprüche 1 bis 3 enthält, wobei die Zusammensetzung keine Oxidationsbase enthält.

**6.** Zusammensetzung nach Anspruch 5, dadurch gekennzeichnet, daß die Verbindung der Formel (I) oder (II) in der Zusammensetzung in Mengenanteilen im Bereich von 0,01 bis 8 Gew.-%, bezogen auf das Gesamtgewicht der Zusammensetzung, vorliegt.

**7.** Zusammensetzung nach Anspruch 6, dadurch gekennzeichnet, daß die Verbindung der Formel (I) oder (II) in der Zusammensetzung in Mengenanteilen im Bereich von 0,03 bis 5 Gew.-%, bezogen auf das Gesamtgewicht der Zusammensetzung, vorliegt.

**8.** Zusammensetzung nach einem der Ansprüche 5 bis 7, dadurch gekennzeichnet, daß das zum Färben geeignete Medium ein wäßriges Medium ist, das aus Wasser oder einem Gemisch aus Wasser/Lösungsmittel(n) besteht.

**9.** Zusammensetzung nach Anspruch 8, dadurch gekennzeichnet, daß die Lösemittel unter Ethanol, Propanol, Isopropanol, *tert.*-Butylalkohol, Ethylenglykol, Ethylenglykolmonomethylether, Ethylenglykolmonoethylether und Ethylenglykolmonobutylether, Ethylenglykolmonoethyletheracetat, Propylenglykol, Propylenglykolmonomethylether, Dipropylenglykolmonomethylether und Methyllactat ausgewählt sind.

**10.** Zusammensetzung nach einem der Ansprüche 5 bis 9, dadurch gekennzeichnet, daß sie mindestens einen Zusatzstoff enthält, der unter den Fettamiden, anionischen, kationischen, nichtionischen oder amphoteren grenzflächenaktiven Stoffen oder deren Gemischen, Verdickungsmitteln, Parfums, Maskierungsmitteln, Trübungsmitteln und Quellmitteln für Keratinfasern und deren Gemischen ausgewählt ist.

**11.** Zusammensetzung nach einem der Ansprüche 5 bis 10, dadurch gekennzeichnet, daß sie einen pH-Wert im Bereich von 3 bis 12 aufweist.

**12.** Verfahren zum Färben von Keratinfasern, dadurch gekennzeichnet, daß auf die Fasern mindestens eine Zusammensetzung nach einem der Ansprüche 5 bis 11 aufgebracht wird, die Zusammensetzung während einer Zeitspanne, die ausreichend ist, um die Färbung entweder an Luft oder mit einem Oxidationssystem zu entwickeln, mit den Fasern in Kontakt gelassen wird und dann gespült wird.

**13.** Verfahren nach Anspruch 12, dadurch gekennzeichnet, daß die Färbung in Kontakt mit Luft ohne Zugabe eines zusätzlichen Oxidationsmittels entwickeln gelassen wird.

**14.** Verfahren nach Anspruch 12, dadurch gekennzeichnet, daß auf die Fasern eine Zusammensetzung (A) aufgetragen wird, die in einem zum Färben geeigneten Medium mindestens eine Verbindung der Formel (I) oder (II) nach

einem der Ansprüche 1 bis 3 enthält, wobei die Farbe mit einem chemischen Oxidationssystem entwickelt wird, das ausgewählt ist unter:

(i) Iodidionen und Wasserstoffperoxid, wobei die Zusammensetzung (A), die die Verbindung der Formel (I) oder (II) enthält, in diesem Fall ferner entweder Iodidionen oder Wasserstoffperoxid enthält und wobei vor oder nach dem Auftragen der Zusammensetzung (A) eine Zusammensetzung (B) aufgebracht wird, die in einem zum Färben geeigneten Medium enthält:
entweder

(a) wenn die Zusammensetzung (A) Iodidionen enthält, Wasserstoffperoxid bei einem pH-Wert im Bereich von 2 bis 12,

oder

(b) wenn die Zusammensetzung (A) Wasserstoffperoxid enthält, Iodidionen bei einem pH-Wert im Bereich von 3 bis 11;

(ii) Nitritionen, wobei nach dem Auftragen der Zusammensetzung (A), die die Verbindung der Formel (I) oder (II) enthält, eine wäßrige Zusammensetzung (B) aufgebracht wird, die einen sauren pH-Wert aufweist, und wobei die Zusammensetzung (A) oder die Zusammensetzung (B) mindestens ein Nitrit enthält;
(iii) Oxidationsmitteln, die unter Wasserstoffperoxid, Periodsäure und ihren wasserlöslichen Salzen, Natriumhypochlorit, Chloramin T, Chloramin B, Kaliumferricyanid, Silberoxid, Fentons Reagenz, Blei(IV)-oxid, Cäsiumsulfat, Ammoniumpersulfat und Alkalichloriten ausgewählt sind; die Oxidationsmittel liegen in der Zusammensetzung (A) vor, die die Verbindung der Formel (I) oder (II) enthält, oder sie werden gleichzeitig oder getrennt davon anschließend mit einer Zusammensetzung (B) aufgebracht, die diese in einem zum Färben geeigneten Medium enthält;
(iv) Metallanionen, die unter den Permanganaten oder Dichromaten ausgewählt sind, wobei diese Oxidationsmittel vor dem Auftragen der Zusammensetzung (A) mit einer wäßrigen Zusammensetzung (B) bei einem pH-Wert von 2 bis 10 aufgebracht werden;
(v) Metallsalzen der Gruppen 3 bis 8 des Periodensystems, wobei die Metallsalze mit einer Zusammensetzung (B) aufgebracht werden, die diese in einem zum Färben geeigneten Medium enthält, und wobei die Zusammensetzung (B) vor oder nach dem Auftragen der Zusammensetzung (A) aufgebracht wird;
(vi) Salzen von Seltenerdmetallen, wobei die Salze von Seltenerdmetallen mit einer Zusammensetzung (B) aufgebracht werden, die diese in einem zum Färben geeigneten Medium enthält, und wobei die Zusammensetzung (B) vor oder nach dem Auftragen der Zusammensetzung (A), die die Verbindung der Formel (I) oder (II) enthält, aufgebracht wird;
(vii) einem Chinonderivat, das unter den o- oder p-Benzochinonen, o- oder p-Benzochinonmonoiminen, o- oder p-Benzochinondiiminen, 1,2- oder 1,4-Naphthochinonen, o- oder p-Benzochinonsulfonimiden, $\alpha,\omega$-Alkylen-bis-1,4-benzochinonen, 1,2-oder 1,4-Naphthochinonmonoiminen oder 1,2- oder 1,4-Naphthochinondiiminen ausgewählt ist, in einer Zusammensetzung (B), die diese in einem zum Färben geeigneten Medium enthält, wobei die Verbindung der Formel (I) oder (II) und die Chinon-derivate so ausgewählt sind, daß die Differenz des Redoxpotentials $\Delta E$ des Redoxpotentials Ei der Verbindung der Formel (I) oder (II), das bei pH 7 in einem Phosphatmedium durch Voltametrie an einer Glaskohlenstoffelektrode bestimmt wird, und des Redoxpotentials Eq des Chinonderivats, das bei pH 7 in einem Phosphatmedium durch Polarographie an einer Quecksilberelektrode bezogen auf eine gesättigte Kalomel-Elektrode bestimmt wird, entspricht:

$$\Delta E = Ei - Eq \leq 320 \text{ Millivolt},$$

wobei die Zusammensetzung (B) vor oder nach dem Auftragen der Zusammensetzung (A), die die Verbindung der Formel (I) oder (II) enthält, aufgebracht wird.

**15.** Verfahren nach Anspruch 14, dadurch gekennzeichnet, daß auf die Keratinfasern mindestens eine Zusammensetzung (A) aufgetragen wird, die in einem zum Färben geeigneten Medium eine Verbindung der Formel (I) oder (II) in Kombination mit Wasserstoffperoxid enthält und einen pH-Wert im Bereich von 2 bis 7 aufweist, wobei vor oder nach dem Auftragen der Zusammensetzung (A) eine Zusammensetzung (B) aufgebracht wird, die in einem zum Färben geeigneten Medium Iodidionen enthält.

**16.** Verfahren nach Anspruch 14 oder 15, dadurch gekennzeichnet, daß die Iodidionen in der Zusammensetzung (A) oder (B) in Mengenanteilen im Bereich von 0,007 bis 4 Gew.-%, ausgedrückt als Ionen $I^-$, bezogen auf das Gesamtgewicht der Zusammensetzung (A) oder (B), vorliegen.

**17.** Verfahren nach Anspruch 14, dadurch gekennzeichnet, daß auf die Keratinfasern eine Zusammensetzung (A), die die Verbindung der Formel (I) oder (II) enthält, und anschließend eine wäßrige, saure Zusammensetzung (B) aufgetragen wird, wobei die Zusammensetzung (A) oder die Zusammensetzung (B) mindestens ein Nitrit enthält, das unter Nitriten von Alkalimetallen, Erdalkalimetallen oder Ammonium oder beliebigen anderen, kosmetisch akzeptablen Kationen, organischen Nitritderivaten und Nitrit-Vektoren, die Nitrite vom oben genannten Typ bilden, ausgewählt ist, wobei die Nitrite in Mengenanteilen im Bereich von 0,02 bis 1 mol/l vorliegen.

**18.** Verfahren nach Anspruch 14, dadurch gekennzeichnet, daß auf die Keratinfasern eine Zusammensetzung (B), die in einem zum Färben geeigneten Medium bei einem pH-Wert von 2 bis 10 ein Metallanion enthält, welches eine hohe Affinität für Keratin aufweist und unter Permanganaten und Dichromaten ausgewählt ist, und in einem zweiten Schritt eine Zusammensetzung (A) aufgetragen wird, die in einem zum Färben geeigneten Medium bei einem pH-Wert von 4 bis 10 eine Verbindung der Formel (I) oder (II) nach Anspruch 1 enthält, wobei die Permanganate und Dichromate in Anionen-Molalitäten über $10^{-3}$ mol/1000g verwendet werden, und dadurch, daß die Zusammensetzungen keine organische Verbindung enthalten, die auf die Anionen reduzierend wirkt.

**19.** Verfahren nach Anspruch 14, dadurch gekennzeichnet, daß auf die Keratinfasern eine Zusammensetzung (A) aufgetragen wird, die in einem zum Färben geeigneten Medium eine Verbindung der Formel (I) oder (II) nach Anspruch 1 enthält, und vor oder nach dem Auftragen der Zusammensetzung (A) eine Zusammensetzung (B) aufgebracht wird, die in einem zum Färben geeigneten Medium ein Metallsalz enthält, das unter den Salzen von Mangan, Cobalt, Eisen, Kupfer und Silber ausgewählt ist, wobei die Metallsalze in Mengenanteilen im Bereich von 0,01 bis 2 Gew.-%, ausgedrückt als Metallionen, bezogen auf das Gesamtgewicht der Zusammensetzung, enthalten sind.

**20.** Verfahren nach Anspruch 14, dadurch gekennzeichnet, daß eine Zusammensetzung (A) aufgetragen wird, die in einem zum Färben geeigneten Medium mindestens eine Verbindung der Formel (I) oder (II) nach Anspruch 1 enthält, und vor oder nach dem Auftragen der Zusammensetzung (A) eine Zusammensetzung (B) aufgebracht wird, die in einem zum Färben geeigneten Medium ein Salz eines Seltenerdmetalls enthält, das unter den Salzen von Cer, Lanthan, Europium, Gadolinium, Ytterbium und Dysprosium ausgewählt ist, wobei die Seltenerdmetallsalze in Mengenanteilen im Bereich von 0,1 bis 8 Gew.-%, bezogen auf das Gesamtgewicht der Zusammensetzung, vorliegen.

**21.** Verfahren nach Anspruch 14, dadurch gekennzeichnet, daß eine Zusammensetzung (A) aufgetragen wird, die in einem zum Färben geeigneten Medium mindestens eine Verbindung der Formel (I) oder (II) nach Anspruch 1 enthält, und vor oder nach dem Auftragen der Zusammensetzung (A) eine Zusammensetzung (B) aufgebracht wird, die in einem zum Färben geeigneten Medium ein Chinonderivat enthält, das unter 1,4-Benzochinon und 2-β-Hydroxyethylthio-1,4-benzochinon ausgewählt ist, wobei das Chinonderivat in Mengenanteilen im Bereich von 0,005 bis 1 mol/l vorliegt.

**22.** Verfahren nach Anspruch 14 bis 16, dadurch gekennzeichnet, daß, wenn als Oxidationsmittel eine Zusammensetzung auf der Basis von Wasserstoffperoxid verwendet wird, der Gehalt an Wasserstoffperoxid in der Zusammensetzung im Bereich von 1 bis 40 Volumina liegt.

**23.** Mittel zum Färben von Keratinfasern und insbesondere von menschlichen Keratinfasern mit mehreren Komponenten, dadurch gekennzeichnet, daß es eine erste Komponente aus einer Zusammensetzung (A) nach einem der Ansprüche 14 bis 22, die eine Verbindung der Formel (I) oder (II) nach Anspruch 1 enthält, und eine zweite Komponente aus einer Zusammensetzung (B) nach einem der Ansprüche 14 bis 22 umfaßt.

**24.** Vorrichtung mit mehreren Abteilungen oder "Kit zum Färben", dadurch gekennzeichnet, das sie eine erste Abteilung, die die Zusammensetzung (A) nach einem der Ansprüche 14 bis 22, die eine Verbindung der Formel (I) oder (II) nach Anspruch 1 enthält, und eine zweite Abteilung aufweist, die die Zusammensetzung (B) nach einem der Ansprüche 14 bis 22 enthält.

## Claims

1. 2-Imino-2,3-dihydro-1H-indole derivatives corresponding to formula (I) or (II) below:

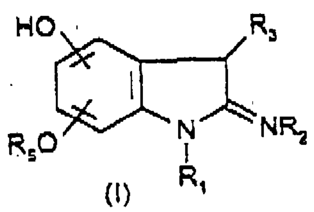

(I)

or

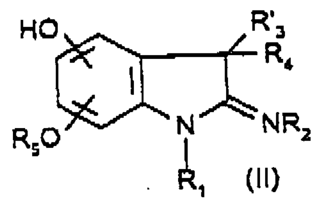

(II)

in which :

- $R_1$, $R_2$ and $R_3$, which may be identical or different, denote a hydrogen atom or a $C_1$-$C_4$ alkyl, carboxyl, ($C_1$-$C_4$)alkoxycarbonyl, $C_1$-$C_4$ monohydroxyalkyl, $C_2$-$C_4$ polyhydroxyalkyl, ($C_1$-$C_4$)alkoxy($C_1$-$C_4$)alkyl, mono($C_1$-$C_4$) alkylamino ($C_1$-$C_4$) alkyl or di ($C_1$-$C_4$) alkylamino ($C_1$-$C_4$)alkyl radical;
- $R'_3$ and $R_4$, which may be identical or different, denote a $C_1$-$C_4$ alkyl, carboxyl, ($C_1$-$C_4$)alkoxycarbonyl, $C_1$-$C_4$ monohydroxyalkyl, $C_2$-$C_4$ polyhydroxyalkyl, ($C_1$-$C_4$)alkoxy ($C_1$-$C_4$)alkyl, mono($C_1$-$C_4$)alkylamino($C_1$-$C_4$)alkyl or di($C_1$-$C_4$)alkylamino ($C_1$-$C_4$)alkyl radical;
- $R_5$ denotes a hydrogen atom or a $C_1$-$C_4$ alkyl, $C_1$-$C_4$ monohydroxyalkyl, $C_2$-$C_4$ polyhydroxyalkyl, ($C_1$-$C_4$)alkoxy ($C_1$-$C_4$)alkyl , mono ($C_1$-$C_4$)alkylamino($C_1$-$C_4$)alkyl or di($C_1$-$C_4$)alkylamino($C_1$-$C_4$) alkyl radical; it being possible for the said alkyl or alkoxy radicals to be linear or branched; as well as the addition salts thereof with an acid;

and the possible tautomeric forms thereof.

2. Derivatives according to Claim 1, characterized in that the addition salts with an acid are chosen from the group consisting of the hydrochlorides, hydrobromides, sulphates, tartrates, acetates and lactates.

3. 5,6-Dihydroxy-1,3-dihydroindol-2-ylideneamine and the addition salts thereof with an acid.

4. Process for the preparation of the compounds of formula (IA) or (IIA) below, or one of the tautomeric forms thereof:

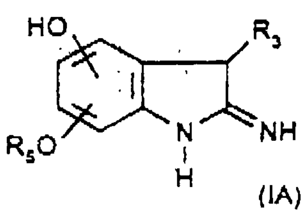

(IA)

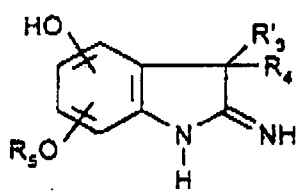

(II A)

in which the radicals $R_3$, $R'_3$, $R_4$ and $R_5$ have the same meanings as those in Claim 1, characterized in that a compound of formula (1) or (1') below:

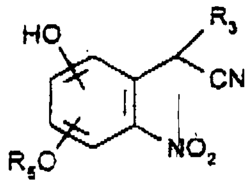 (1) 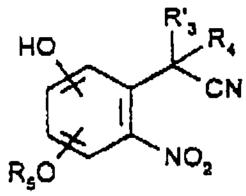 (1')

in which the meanings of the radicals $R_3$, $R'_3$, $R_4$ and $R_5$ are identical to those indicated for formula (I) or (II) of Claim 1, is reacted by carrying out either a chemical reduction in the presence of an organic solvent using metals, or a selective hydrogenation reaction using a catalyst, in order to obtain a compound of formula (2) or (2') below:

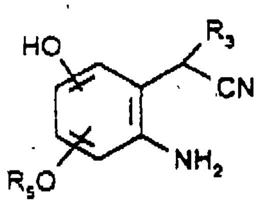

(2)

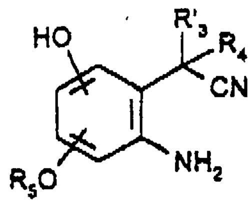

(2')

in which the radicals $R_3$, $R'_3$, $R_4$ and $R_5$ have the same meanings as those indicated in Claim 1;
after which a cyclization reaction is carried out in acidic medium in the presence of an organic solvent.

**5.** Dye composition intended to be used to dye keratin fibres, characterized in that it contains, in a medium which is suitable for dyeing, at least one compound of formula (I) or (II), or at least one of the addition salts thereof with an acid, as defined according to any one of Claims 1 to 3, the said composition being free from oxidation base.

**6.** Composition according to Claim 5, characterized in that the compound of formula (I) or (II) is present in the composition in proportions of between 0.01 and 8% by weight relative to the total weight of the composition.

**7.** Composition according to Claim 6, characterized in that the compound of formula (I) or (II) is present in the composition in proportions of between 0.03 and 5% by weight relative to the total weight of the composition.

**8.** Composition according to any one of Claims 5 to 7, characterized in that the medium which is suitable for dyeing is an aqueous medium consisting of water or a water/solvent(s) mixture.

**9.** Composition according to Claim 8, characterized in that the solvents are chosen from ethyl alcohol, propyl alcohol or isopropyl alcohol, tert-butyl alcohol, ethylene glycol, ethylene glycol monomethyl, monoethyl and monobutyl ethers, ethylene glycol monoethyl ether acetate, propylene glycol, propylene glycol and dipropylene glycol monomethyl ethers, and methyl lactate.

**10.** Composition according to any one of Claims 5 to 9, characterized in that it contains at least one adjuvant chosen from fatty amides, anionic, cationic, nonionic or amphoteric surfactants or mixtures thereof, thickeners, fragrances, sequestering agents, opacifiers and agents for swelling keratin fibres, and mixtures thereof.

**11.** Composition according to any one of Claims 5 to 10, characterized in that it has a pH of between 3 and 12.

**12.** Process for dyeing keratin fibres, characterized in that at least one composition as defined in any one of Claims 5 to 11 is applied to these fibres, this composition is kept in contact with the fibres for a period which is sufficient to develop a coloration, either in air or using an oxidizing system, and rinsing is then carried out.

**13.** Process according to Claim 12, characterized in that the coloration is allowed to develop in contact with the air without adding an external oxidizing agent.

**14.** Process according to Claim 12, characterized in that a composition (A) containing, in a medium which is suitable for dyeing, at least one compound of formula (I) or (II) as defined in any one of Claims 1 to 3 is applied to the fibres, the colour being developed using a chemical oxidizing system consisting of:

(i) iodide ions and hydrogen peroxide, the composition (A) containing the compound of formula (I) or (II) also comprising, in this case, either iodide ions or hydrogen peroxide, and application of the composition (A) being preceded or followed by the application of a composition (B) which contains, in a medium which is suitable for dyeing, either:

(a) hydrogen peroxide at a pH of between 2 and 12, when the composition (A) contains iodide ions, or:
(b) iodide ions at a pH of between 3 and 11 when the composition (A) contains hydrogen peroxide;

(ii) nitrite ions, application of the composition (A) containing the compound of formula (I) or (II) being followed by the application of an aqueous composition (B) having an acidic pH, the composition (A) or the composition (B) containing at least one nitrite;
(iii) oxidizing agents chosen from hydrogen peroxide, periodic acid and its water-soluble salts, sodium hypochlorite, chloramine T, chloramine B, potassium ferricyanide, silver oxide, Fenton's reagent, lead (IV) oxide, caesium sulphate, ammonium persulphate and alkali metal chlorites; these oxidizing agents being present in composition (A) containing the compound of formula (I) or (II) or applied, simultaneously or sequentially, by means of a composition (B) containing them in a medium which is suitable for dyeing;
(iv) anions of a metal chosen from the permanganates or dichromates, these oxidizing agents being applied by means of an aqueous composition (B), at a pH of from 2 to 10, before the composition (A) is applied;
(v) salts of metals from groups 3 to 8 of the Periodic Table, these metal salts being applied by means of a composition (B) containing them in a medium which is suitable for dyeing, the composition (B) being applied before or after the composition (A) is applied;
(vi) rare-earth metal salts, these rare-earth metal salts being applied by means of a composition (B) containing them in a medium which is suitable for dyeing, the composition (B) being applied before or after the composition (A) containing the compound of formula (I) or (II) is applied;
(vii) a quinone derivative chosen from ortho- or para-benzoquinones, monoimine or diimine ortho- or para-benzoquinones, 1,2- or 1,4-naphthoquinones, sulphonimide ortho- or para-benzoquinones, $\alpha$, $\omega$-alkylenebis-1,4-benzoquinones or monoimine or diimine 1,2- or 1,4-naphthoquinones by means of a composition (B) containing them in a medium which is suitable for dyeing, the compound of formula (I) and the quinone derivatives being chosen such that the redox potential difference $\Delta E$ between the redox potential Ei of the compound of formula (I) or (II) determined at pH 7 in phosphate medium on a glass carbon electrode by voltammetry and the redox potential Eq of the quinone derivative determined at pH 7 in phosphate medium by polarography on a mercury electrode relative to the saturated calomel electrode, is such that:

$$\Delta E = Ei - Eq \leq 320 \text{ millivolts};$$

the composition (B) being applied before or after the composition (A) containing the compound of formula (I) or (II) is applied.

**15.** Process according to Claim 14, characterized in that at least one composition (A) is applied to the keratin fibres, this composition containing, in a medium which is suitable for dyeing, a compound of formula (I) or (II) in combination with hydrogen peroxide and having a pH of between 2 and 7, application of the composition (A) being preceded or followed by the application of a composition (B) which contains iodide ions in a medium which is suitable for dyeing.

**16.** Process according to Claim 14 or 15, characterized in that the iodide ions are present in the composition (A) or (B) in proportions of between 0,007 and 4% by weight, expressed as ions $I^-$, relative to the total weight of the composition (A) or (B).

**17.** Process according to Claim 14, characterized in that a composition (A) containing the compound of formula (I) or (II) is applied to the keratin substances and an aqueous acidic composition (B) is then applied, composition (A) or composition (B) containing at least one nitrite chosen from the nitrites of alkali metals, of alkaline-earth metals or of ammonium or of any other cosmetically acceptable cation, an organic nitrite derivative and nitrite vectors which generate a nitrite of the type defined above, these nitrites being present in proportions of between 0.02 and 1 mol/litre.

18. Process according to Claim 14, characterized in that a composition (B) is applied to the keratin fibres, this composition containing, in a medium which is suitable for dyeing, at a pH of between 2 and 10, an anion of a metal having good affinity for keratin, chosen from permanganates and dichromates, and in that, in a second stage, a composition (A) is applied containing, in a medium which is suitable for dyeing, at a pH of between 4 and 10, a compound of formula (I) or (II) as defined in Claim 1; these permanganates or dichromates being used in anion molalities of greater than $10^{-3}$ mol/1000 g and in that the compositions contain no organic agents having a reducing effect on the anions.

19. Process according to Claim 14, characterized in that a composition (A) is applied to the keratin fibres, this composition containing, in a medium which is suitable for dyeing, a compound of formula (I) or (II) as defined in Claim 1 and in that a composition (B) containing, in a medium which is suitable for dyeing, a metal salt chosen from manganese, cobalt, iron, copper and silver salts, these metal salts being used in proportions of between 0.01 and 2% by weight, expressed as metal ions, relative to the total weight of the composition, is applied before or after composition (A).

20. Process according to Claim 14, characterized in that a composition (A) is applied containing, in a medium which is suitable for dyeing, at least one compound of formula (I) or (II) as defined in Claim 1 and in that, before or after this composition, a composition (B) is applied containing, in a medium which is suitable for dyeing, a rare-earth metal salt chosen from cerium, lanthanum, europium, gadolinium, ytterbium and dysprosium salts, these rare-earth metal salts being present in proportions of between 0.1 and 8% by weight relative to the total weight of the composition.

21. Process according to Claim 14, characterized in that a composition (A) is applied, containing, in a medium which is suitable for dyeing, at least one compound of formula (I) or (II) as defined in Claim 1 and in that, before or after this composition, a composition (B) is applied containing, in a suitable medium, a quinone derivative chosen from 1,4-benzoquinone and 2-β-hydroxyethylthio-1,4-benzoquinone, this quinone derivative being present in proportions of between 0.005 and 1 mol/litre.

22. Process according to Claims 14 to 16, characterized in that, when a composition based on hydrogen peroxide is used as oxidizing means, the hydrogen peroxide content in the composition is between 1 and 40 volumes.

23. Multi-component agent for dyeing keratin fibres, in particular human keratin fibres, characterized in that it comprises a first component consisting of a composition (A) as defined in any one of Claims 14 to 22 containing a compound of formula (I) or (II) as defined in Claim 1, and a second component consisting of one of the compositions (B) defined in any one of Claims 14 to 22.

24. Multi-compartment device or "dyeing kit", characterized in that it comprises a first compartment containing a composition (A) as defined in any one of Claims 14 to 22 containing a compound of formula (I) or (II) as defined in Claim 1, and a second compartment containing a composition (B) as defined in any one of Claims 14 to 22.